# EUROPEAN PATENT APPLICATION

(11) **EP 1 930 324 A1**
(43) Date of publication of application: **11.06.2008**
(21) Application number: 06024597.4
(22) Date of filing: 28.11.2006
(51) Int. Cl.: C07D 213/42

(54) **Process for the preparation of atazanavir**

(71) Applicant: SOLMAG S.p.A., 26837 Mulazzano LO (IT)
(72) Inventor: Pizzocaro, Roberta, 20024 Garbagnate Milanese (IT); Galimi Stefania, 20024 Garbagnate Milanese (IT); Rossi Alessia, 20024 Garbagnate Milanese (IT)
(74) Representative: Minoja, Fabrizio

(57) **Abstract**

The invention relates to a process for the preparation of Atazanavir, which comprises reacting the hydrochloride salt of compound (6) 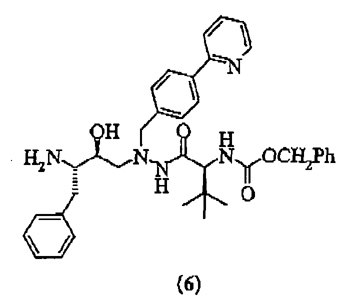 with N-methoxycaxbonyl-L-*tert*-leucine, the removal of the benzyloxycarbonyl group and the reaction with methoxycarbonyl chloride. The process is particularly advantageous in that it allows to use reduced amounts of N-methoxycarbonyl-L-*tert*-leucine and avoids the use of unstable intermediates.

## Description

### Field of the invention

The present invention relates to a process for the preparation of Atazanavir.

### Background of the invention

Atazanavir (**I**) ([3S-(3R*,8R*,9R*,12R*)]-3,12-bis(1,1-dimethylethyl)-8-hydroxy-4,11-dioxo-9-(phenylmethyl)-6-[[4-(2-pyridinyl)phenyl]methyl]-2,5,6,10,13-pentaazatetradecanedioic acid dimethyl ester) 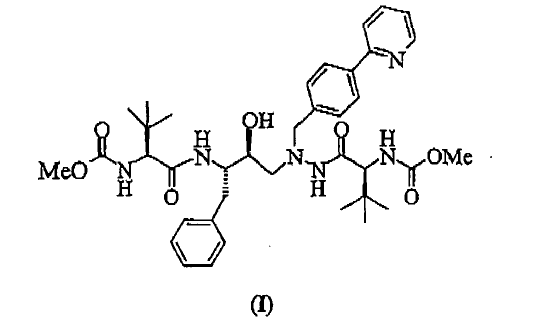 is a known antiviral agent and anti-HIV protease inhibitor, disclosed in EP 0 900 210 and in its equivalent US 5,849,911. These patents disclose preparation methods based on different synthetic approaches and intermediates; some of them are schematically represented and discussed below:

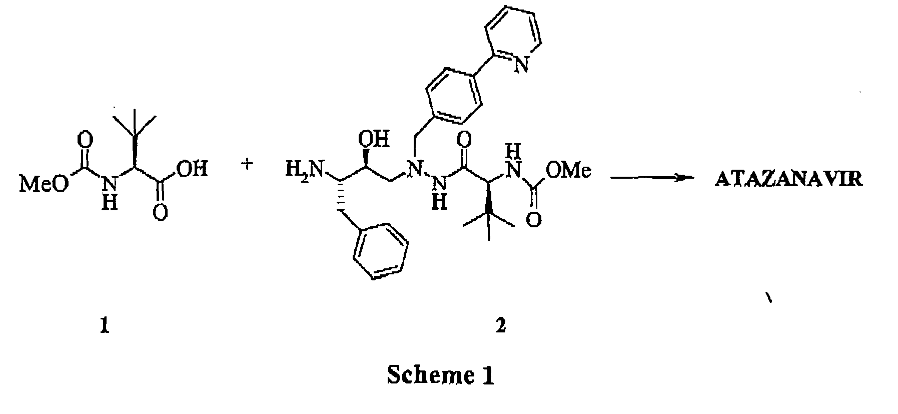

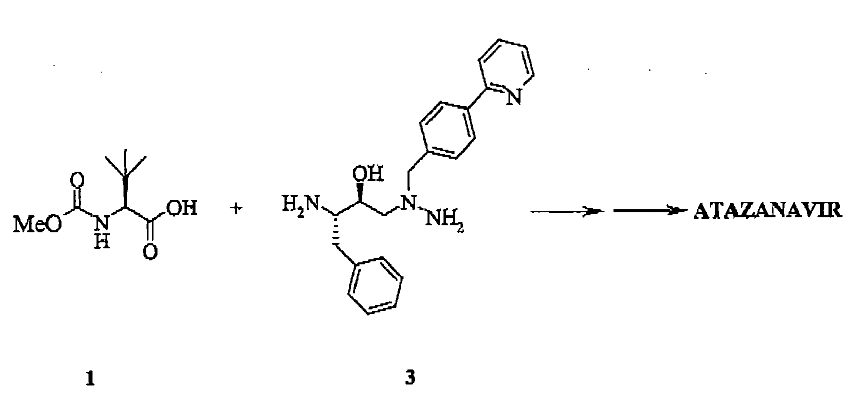

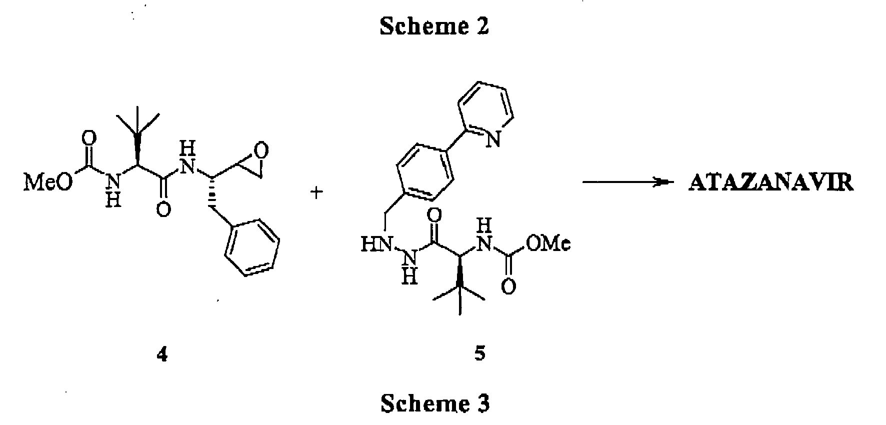

In the processes of schemes 1 and 2 N-methoxycarbonyl-L-*tert*-leucine **(1)** is reacted with amino derivatives **(2)** and **(3)** respectively, while in scheme 3 epoxide **(4)** is reacted with hydrazine **(5).**

These processes suffer from some drawbacks and are difficult to carry out on an industrial scale. In particular, N-methoxycarbonyl-L-*tert*-leucine is very expensive; the synthesis of compounds **(2)** and **(5)** (schemes 1 and 3) is troublesome, with selectivity issues between the amino groups and compound **(4)** of scheme 3 is poorly stable.

The process of scheme 2 requires the use of a large excess of N-methoxycarbonyl-L-*tert*-leucine (at least three equivalents); moreover, compound **(3)** is difficult to isolate and handle, and highly hygroscopic.

There is still therefore the need for an improved process for the synthesis of Atazanavir.

### Disclosure of the invention

The present invention relates to a process for the preparation of Atazanavir (**I**) 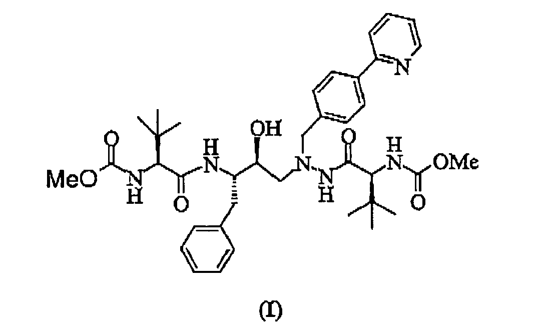 comprising the following steps:
a) reaction of the hydrochloride salt of compound **(6)** 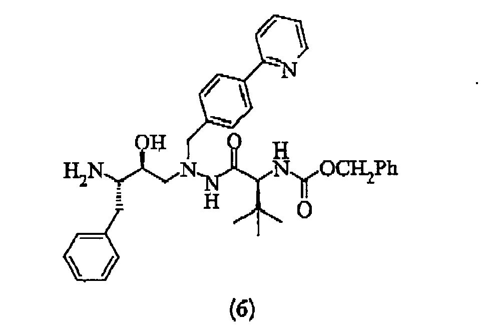 with N-methoxycarbonyl-L-*tert*-leucine **(1)** 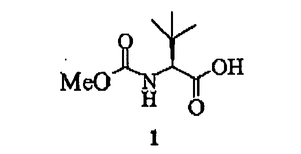 to give compound **(7)** 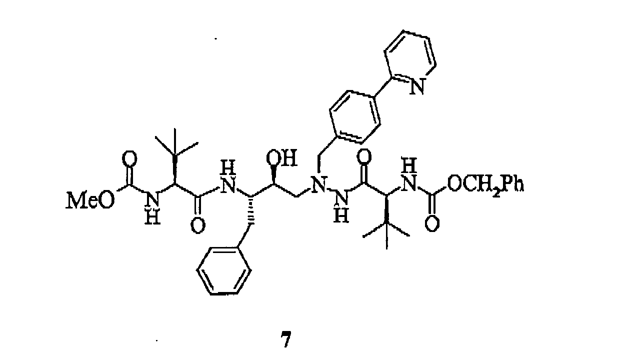
b) removal of the benzyloxycarbonyl group to give compound **(8)** 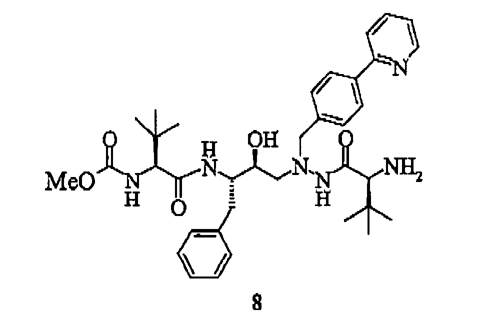 and
c) treatment of compound **8** with methoxycarbonyl chloride to give Atazanavir **(I).**

Step a) is usually carried out in a mixture of a chlorinated solvent and an aprotic polar solvent, preferably selected from methylene chloride, chloroform and dimethylformamide, dimetylacetamide, DMPU, more preferably methylene chloride and dimethylformamide, in the presence of a condensing agent such as DCC, at a temperature of about 0°C, preferably between 0 and 5°C.

Step b) is usually carried out in methanol and ammonium formate and Pd/C as catalyst, while step c) is carried out in a chlorinated solvent, preferably methylene chloride.

The compound of formula **(6)** can be prepared by reacting the hydrazine of formula **(9)** 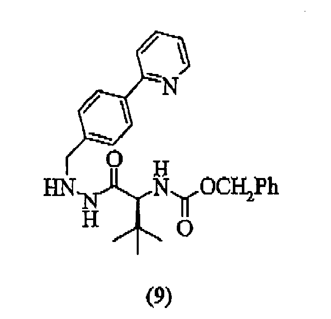 either with epoxide **(10)** 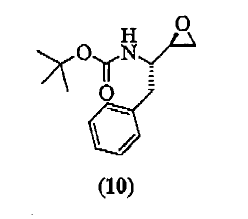 or with epichloridrine **(11)** 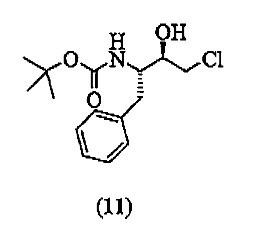 followed by acid treatment to remove the tBoc group. Compound **(6)** is further treated with hydrochloric acid to give the hydrochloride salt. Usually, the removal of the tBoc group is carried out with an amount of hydrochloric acid also sufficient to form the hydrochloride salt of compound **(6).**

The process of the invention is advantageous over the above-discussed prior art in that it avoids the use of amino-derivative **(3)** and it requires lower amounts of N-methoxycarbonyl-L-*tert*-leucine.

Moreover, the removal of the benzyloxycarbonyl (Cbz) group is a clean reaction and compound **(6),** its precursors and compounds **(7)** and **(8)** can be isolated in the crystalline form, which makes their work-up easier.

Compound **(6)** is novel and is a further object of the invention.

The invention will be now illustrated in greater detail by means of some examples.

### EXAMPLES

### Example 1 - Preparation of compound (9)

In a 250 mL round bottom flask a solution of Cbz-tert-leucine (12.3 g) in CH₂Cl₂ (45 mL) was added with 7.9 g of HOBt (1-hydroxy 1H-benzotriazole) in 27 ml of DMF. The mixture was cooled to 0°-5°C, then added with 10.1 g of DCC dissolved in 10 ml of CH₂Cl₂, keeping the temperature below 10°C; after about one hour at this temperature, the suspension was filtered and the solid was washed on the filter with small amounts of CH₂Cl₂. The filtrate was transferred into a one-liter round bottom flask and added with a solution of 33,6 g of K₂HPO_{4'}3H₂O in 45 ml water at 0°-5°C, then 4-(pyridin-2-yl)benzylhydrazine was added keeping the temperature below 10°C and the resulting mixture was allowed to stand overnight. The suspension was filtered and the organic layer was washed with water, with a solution of 27, 5 g of NaH₂PO₄-H₂O in 45 ml water, and again with water, then separated and stirred with a solution of 12.1 g of 30% sodium hydroxide in 45 ml water. Thereafter, the sodium hydroxide solution was discharged and the organic layer was washed in sequence with water, a sodium hydrogen phosphate solution (the same as above), water and brine, then separated and dried over sodium sulphate. After evaporation of the solvent under vacuum the title compound (18.5 g) was obtained as a yellowish solid.

### Example 2 - Preparation of t-Boc-protected compound (6)

In a 250 ml round bottom flask 11,3 g of epoxide **(10),** and 18.5 g of compound **(9),** prepared as described in example 1, were heated at 80°C for 48 hours in toluene (110 ml). The reaction mixture was then cooled and filtered. The solid was washed with toluene and dried to give 13.8 g of an off-white product.

### Example 3 - Preparation of compound (6) hydrochloride salt

In a round bottom flask 13.8 g of the compound of example 2, were dissolved in THF (45 mL) and 15 mL of water. The solution was cooled to 0°-5°C, added with 37% HCI maintaining the temperature below 10°C, then heated at 30°C for 16 hours; after this time the mixture was cooled to room temperature and the organic solvent was evaporated *in vacuo.* The resulting solution was stirred and added first with ethyl acetate (200 mL), then with 200 mL of NaHCO₃ saturated solution. The aqueous phase was discharged and the organic phase was dried over anhydrous sodium sulphate, filtered, and evaporated *in vacuo.* 12.2 g of the hydrochloride salt was obtained as a yellowish solid, which was used without further purifications for the following step.

### Example 4 - Preparation of compound (7)

In a round bottom flask methoxycarbonyl-*tert*-leucine **(1)** (4.16) g was dissolved in CH₂Cl₂ (40 mL) and DMF (10 mL). This solution was added with 3.42 g of HOBt, cooled to 0-5°C and added again with 4.54 g of DCC in 20 mL of CH₂Cl₂. The resulting suspension was stirred at 0-5°C for about one hour, then added with a suspension of (6) hydrochloride salt (12.2 g) in CH₂Cl₂ (20 mL) and stirred at 0°C overnight. The solid was filtered off and the filtrate was evaporated *in vacuo* to an oily residue which was taken-up with 100 mL of EtOAc; the precipitated solid was filtered off. The filtrate was washed twice with a 5% aqueous solution of NaHCO₃, and the organic phase was separated, dried over anhydrous sodium sulphate and evaporated *in vacuo,* to obtain a solid residue which was taken up with acetone. The resulting suspension was heated to 60°C for about 10 minutes, then cooled to room temperature to precipitate a white solid which was filtered and washed with acetone on the filter. The filtrate was evaporated in vacuo, and this procedure (taking up with acetone/heating, cooling and filtering) was repeated twice, thereby obtaining 12.2 g of the title compound as a white solid.

### Example 5 - Preparation of compound (8)

Compound **7** (12.2 g), obtained as described in example **4,** was suspended in methanol (90 mL), then added with 1.33 g of Pd-C 5% and the suspension was heated to reflux. The mixture was then cooled to 40°C, and a solution of 4.93 g of ammonium formate in methanol/water was added over about one hour. The reaction was heated at 40°C for about one hour, then cooled to about 25-30°C. The suspension was filtered and the filtrate was evaporated in vacuo to a solid residue, which was added with 100 mL of EtOAc and washed with a 5% aqueous solution of NaHCO₃. The organic phase was dried over anhydrous sodium sulphate, filtered, and evaporated *in vacuo* to yield 9.47 of compound **8** as a white solid, which was used for the next step without further purification.

### Example 6 - Preparation of Atazanavir

Compound **(8)** (9.47 g), obtained according to example 5, was suspended in 200 mL of CH₂Cl₂, added with 1.99 g of diisopropyl ethylamine, and the resulting mixture was cooled to about 0°C. 1.45 g of methyl chloroformate in 25 mL of CH₂Cl₂ were added dropwise over about 2 hours. The suspension was then stirred for about half an hour at 0°C and added with an aqueous solution of 10% NaHCO₃ (100 mL) at 0°C with vigorous stirring. The organic phase was the separated, dried over anhydrous sodium sulphate, filtered and evaporated *in vacuo* to afford 8.46 g of a white solid, which was hot-crystallised from ethanol (65 mL) and water (33 mL). The resulting solid was filtered, washed on the filter with an ethanol/water mixture and dried, to obtain 5.1.g of Atazanavir as a white solid.

## Claims

1. Process for the preparation of Atazanavir **(I)** 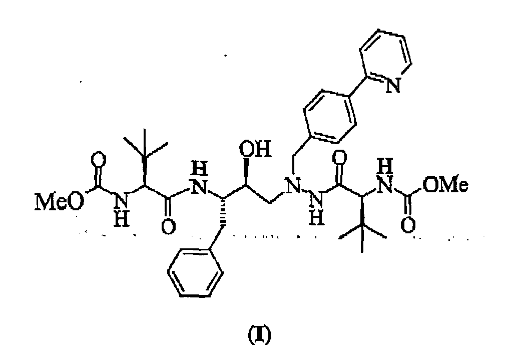 comprising the following steps:
a) reaction of the hydrochloride salt of compound **(6)** 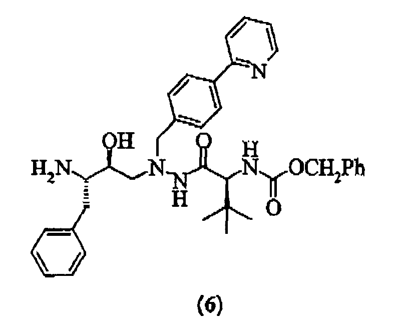 with N-methoxycarbonyl-L-*tert*-leucine **(1)** 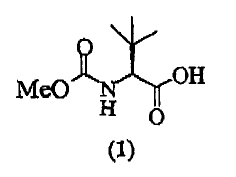 to give compound **(7)** 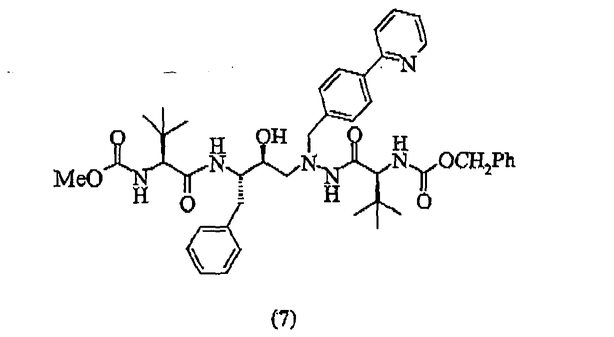
b) removal of the benzyloxycarbonyl group to give compound **(8)** 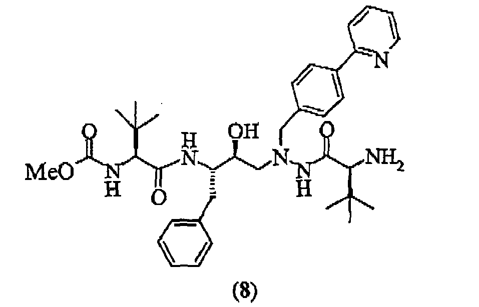 and
c) treatment of compound **(8)** with methoxycarbonyl chloride to give
Atazanavir.

2. The process of claim 1 wherein the hydrochloride salt of the compound of formula (6) is prepared by reacting hydrazine of formula **(9)** 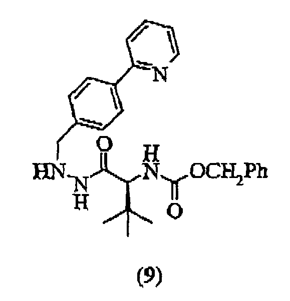 either with epoxide **(10)** 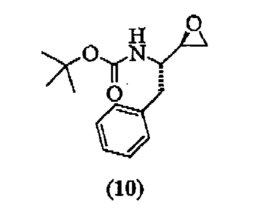 or with epichloridrine **(11)** 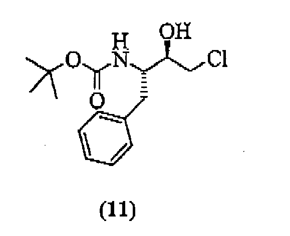 followed by acid treatment with hydrochloric acid.

3. The compound of formula **(6)** 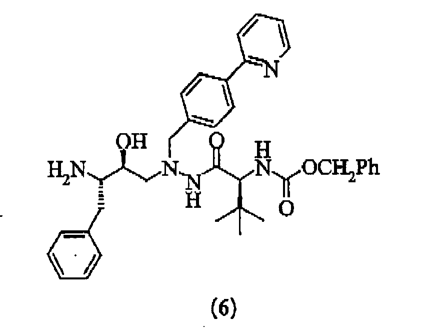 as an intermediate for the preparation of Atazanavir.
